(19) Europäisches Patentamt
European Patent Office
Office européen des brevets

(11) EP 3 728 377 B1

(12) # EUROPÄISCHE PATENTSCHRIFT

(45) Veröffentlichungstag und Bekanntmachung des Hinweises auf die Patenterteilung:
**12.10.2022 Patentblatt 2022/41**

(21) Anmeldenummer: **18815709.3**

(22) Anmeldetag: **12.12.2018**

(51) Internationale Patentklassifikation (IPC):
***C08G 18/50** (2006.01)* ***C08G 18/66** (2006.01)*
***C08G 18/76** (2006.01)* ***C08J 9/14** (2006.01)*
***C08G 18/09** (2006.01)* ***C08G 18/18** (2006.01)*
***C08G 18/22** (2006.01)* ***C08G 18/38** (2006.01)*
***C08G 18/40** (2006.01)* ***C08G 18/42** (2006.01)*
***C07C 269/04** (2006.01)* ***C07C 271/20** (2006.01)*

(52) Gemeinsame Patentklassifikation (CPC):
(C-Sets verfügbar)
**C08G 18/3831; B32B 5/18; B32B 5/20; B32B 5/245; B32B 15/046; B32B 15/12; B32B 15/20; B32B 19/047; B32B 21/02; B32B 21/047; B32B 29/007; B32B 29/02; C07C 269/04; C07C 271/20; C08G 18/092;** (Forts.)

(86) Internationale Anmeldenummer:
**PCT/EP2018/084509**

(87) Internationale Veröffentlichungsnummer:
**WO 2019/121215 (27.06.2019 Gazette 2019/26)**

(54) **FLAMMGESCHÜTZTE POLYURETHAN-HARTSCHAUMSTOFFE**

FLAME RETARDANT RIGID POLYURETHANE FOAMS

MOUSSE RIGIDE DE POLYURÉTHANE IGNIFUGÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2017 EP 17207933**

(43) Veröffentlichungstag der Anmeldung:
**28.10.2020 Patentblatt 2020/44**

(73) Patentinhaber: **Covestro Intellectual Property GmbH & Co. KG**
**51373 Leverkusen (DE)**

(72) Erfinder:
- **NEFZGER, Hartmut**
  **50259 Pulheim (DE)**
- **HILKEN, Persefoni**
  **50937 Köln (DE)**
- **MEURESCH, Markus**
  **51109 Köln (DE)**
- **WOLF, Aurel**
  **42489 Wülfrath (DE)**

(74) Vertreter: **Levpat**
**c/o Covestro AG**
**Gebäude 4825**
**51365 Leverkusen (DE)**

(56) Entgegenhaltungen:
**EP-A1- 1 548 001** **EP-A1- 3 098 251**
**EP-A2- 1 571 167** **WO-A1-03/066580**
**WO-A2-2015/138684** **DE-A1- 2 731 012**
**US-A1- 2006 160 979** **US-B2- 8 097 741**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 728 377 B1

(52) Gemeinsame Patentklassifikation (CPC): (Forts.)
**C08G 18/1816; C08G 18/225; C08G 18/4018; C08G 18/4211; C08G 18/4238; C08G 18/425; C08G 18/5021; C08G 18/66; C08G 18/7664; C08J 9/0014; C08J 9/0019; C08J 9/0023; C08J 9/0038; C08J 9/0061; C08J 9/141; C08K 5/521;** B32B 2266/0278; B32B 2307/102; B32B 2307/3065; B32B 2307/72; B32B 2419/00; C08G 2110/0025; C08G 2110/005; C08J 2201/022; C08J 2203/14; C08J 2205/052; C08J 2205/10; C08J 2375/04; C08J 2483/12

C-Sets
**C08K 5/521, C08L 75/04**

## Beschreibung

Die vorliegende Erfindung betrifft flammgeschützte Polyurethan-Hartschaumstoffe bzw. Polyurethan/Polyisocyanurat-Hartschaumstoffe (im Folgenden einzeln oder gemeinsam auch als "PUR-/PIR-Hartschaumstoffe" bezeichnet) mit Tri-Urethan-Triolen (im Folgenden auch als "TUT" bezeichnet), sowie Verfahren zu ihrer Herstellung und die Verwendung von Tri-Urethan-Triolen in Systemen zur Herstellung von PUR-/PIR-Hartschaumstoffen und Mischungen enthaltend ein Tri-Urethan-Triol und eine Verbindung ausgewählt aus der Gruppe bestehend aus Polyetherpolyol, Polyesterpolyol, Polyethercarbonatpolyol und Polyetheresterpolyol.

Die Herstellung von Polyethercarbonatpolyolen durch katalytische Umsetzung von Alkylenoxiden (Epoxiden) und Kohlendioxid in Anwesenheit von H-fünktionellen Startersubstanzen ("Starter") wird seit mehr als 40 Jahren intensiv untersucht (z. B. Inoue et al., Copolymerization of Carbon Dioxide and Epoxide with Organometallic Compounds; Die Makromolekulare Chemie 130, 210-220, 1969). Diese Reaktion ist in Schema (I) schematisch dargestellt, wobei R für einen organischen Rest wie Alkyl, Alkylaryl oder Aryl steht, der jeweils auch Heteroatome wie beispielsweise O, S, Si usw. enthalten kann, und wobei e, f und g für eine ganzzahlige Zahl stehen, und wobei das hier im Schema (I) gezeigte Produkt für das Polyethercarbonatpolyol lediglich so verstanden werden soll, dass sich Blöcke mit der gezeigten Struktur im erhaltenen Polyethercarbonatpolyol prinzipiell wiederfinden können, die Reihenfolge, Anzahl und Länge der Blöcke sowie die OH-Funktionalität des Starters aber variieren kann und nicht auf das in Schema (I) gezeigte Polyethercarbonatpolyol beschränkt ist. Diese Reaktion (siehe Schema (I)) ist ökologisch sehr vorteilhaft, da diese Reaktion die Umsetzung eines Treibhausgases wie $CO_2$ zu einem Polymer darstellt und so eine entsprechende Menge Alkylenoxid ersetzt, d.h. einspart. Als Nebenprodukt, entsteht das in Schema (I) gezeigte cyclische Carbonat (beispielsweise für R = $CH_3$ Propylencarbonat, im Folgenden auch als cPC bezeichnet, oder für R = H Ethylencarbonat, im Folgenden auch als cEC bezeichnet).

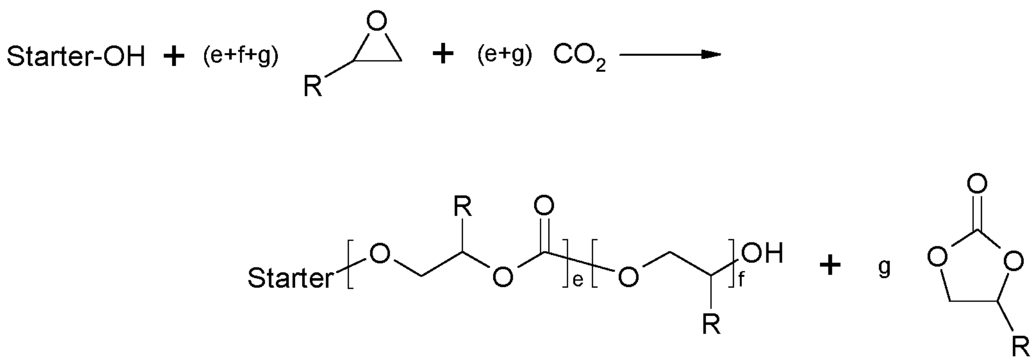
(I)

Für die Herstellung derartiger Polyethercarbonatpolyole haben sich Doppelmetallcyanid (DMC)-Katalysatoren als besonders vorteilhaft erwiesen.

In EP 3 098 251 A1 wird ein Verfahren zur Herstellung von Diurethandiolen aus cyclischen Carbonaten und Diaminen offenbart. Die erhaltenen Diurethandiole werden als H-funktionelle Starterverbindung zur Herstellung von Polyetherpolyolen eingesetzt. Des Weiteren wird ein Verfahren zur Herstellung von Polyurethan-Weichschaumstoffen aus den erhaltenen Polyetherpolyolen offenbart. Es werden jedoch weder Tri-Urethan-Triole offenbart, noch der Einsatz von Diurethandiolen als Isocyanatreaktive Komponente offenbart.

Die der vorliegenden Erfindung zu Grunde liegende Aufgabe bestand deshalb darin, das in der Herstellung von Polyethercarbonatpolyolen als Nebenprodukt anfallende cyclische Carbonat für die Herstellung von flammgeschützten Polyurethan-Hartschaumstoffen stofflich zu verwerten.

Eine weitere Aufgabe bestand darin, den Flammschutz von Polyurethan-Hartschaumstoffen zu verbessern und den Anteil von halogenierten Flammschutzmitteln in der Herstellung von Polyurethan-Hartschaumstoffen zu verringern.

Diese Aufgabe wird erfindungsgemäß gelöst durch ein Verfahren zur Herstellung von PUR-/PIR-Hartschaumstoffen, enthaltend

**A1** eine Isocyanatreaktive Komponente

**A2** Flammschutzmittel

**A3** Treibmittel

**A4** Katalysator

**A5** gegebenenfalls Hilfs- und Zusatzstoffe

**B** eine organische Polyisocyanatkomponente

dadurch gekennzeichnet, dass

die Isocyanatreaktive Komponente **A1** ein Tri-Urethan-Triol **A1.1** und eine Verbindung **A1.2** ausgewählt aus der Gruppe bestehend aus Polyetherpolyol, Polyesterpolyol, Polyethercarbonatpolyol und Polyetheresterpolyol enthält, wobei das Tri-Urethan-Triol A1.1 eine Struktur gemäß Formel (II) aufweist,

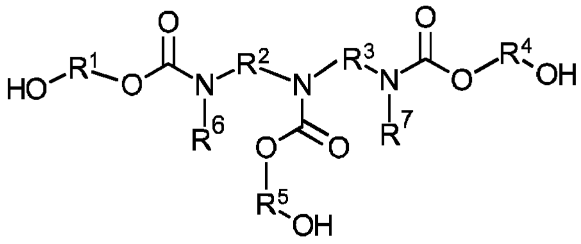

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^2$ bedeutet lineares oder verzweigtes $C_2$ bis $C_{24}$ - Alkylen, $C_3$ bis $C_{24}$ - Cycloalkylen, $C_4$ bis $C_{24}$-Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^3$ bedeutet lineares oder verzweigtes $C_2$ bis $C_{24}$ - Alkylen, $C_3$ bis $C_{24}$ - Cycloalkylen, $C_4$ bis $C_{24}$-Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei $R^1$ bis $R^7$ identisch oder voneinander verschieden sein können..

Überraschend wurde gefunden, dass eine Mischung enthaltend ein Tri-Urethan-Triol **A1.1** gemäß Formel (II) und eine Verbindung **A1.2,** dadurch gekennzeichnet dass, die Verbindung **A1.2** ausgewählt ist aus der Gruppe bestehend aus Polyetherpolyol, Polyesterpolyol, Polyethercarbonatpolyol und Polyetheresterpolyol, in einem Verfahren zur Herstellung von PUR-/PIR-Hartschaumstoffen den Flammschutz der erhaltenen PUR-/PIR-Hartschaumstoffe erhöht.

Die Verwendung des Worts *ein* im Zusammenhang mit zählbaren Größen ist hierbei und im Folgenden nur dann als Zahlwort zu verstehen, wenn dies aus dem Zusammenhang hervorgeht (bspw. durch die Formulierung "*genau ein* "). Ansonsten umfassen Ausdrücke wie "ein Alkylenoxid", "ein Tri-Urethan-Triol **A1.1**" immer auch solche Ausführungsformen, in denen zwei oder mehr Alkylenoxide, zwei oder mehr Tri-Urethan-Triole **A1.1**, eingesetzt werden.

Unter Äquivalentmolmasse ist die durch die Zahl der aktiven Wasserstoffatome geteilte Gesamtmasse des aktive Wasserstoffatome enthaltenden Materials zu verstehen. Im Falle von hydroxygruppenhaltigen Materialien steht sie in folgender Beziehung zur OH-Zahl:

$$\text{Äquivalentmolmasse} = 56100 / \text{OH-Zahl [mg KOH/g]}$$

Die Äquivalentmolmasse steht mit der Molmasse in folgender Beziehung:

$$\text{Molmasse} = \text{Äquivalentmolmasse} * \text{Funktionalität}$$

Mit Funktionalität wird die Anzahl an aktiven Wasserstoffatomen pro Einzelmolekül bezeichnet. Im Rahmen der vorliegenden Erfindung handelt es sich bei den Begriffen Äquivalentmolmasse, Molmasse und Funktionalität um zahlenmittlere Größen.

Die OH-Zahl (auch: Hydroxylzahl) gibt im Falle eines einzelnen zugesetzten Polyols dessen OH-Zahl an. Angaben der OH-Zahl für Mischungen beziehen sich auf die zahlenmittlere OH-Zahl der Mischung, berechnet aus den OH-Zahlen der einzelnen Komponenten in ihren jeweiligen molaren Anteilen. Die OH-Zahl gibt die Menge an Kaliumhydroxid in Milligramm an, welche der bei einer Acetylierung von einem Gramm Substanz gebundenen Menge Essigsäure gleichwertig ist. Die OH-Zahl wird im Rahmen der vorliegenden Erfindung nach der Norm DIN 53240-1 (Juni 2013) bestimmt.

Nachfolgend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

Erfindungsgemäß enthält die Isocyanat-reaktive Komponente **A1** ein Tri-Urethan-Triol **A1.1** gemäß Formel (II) und eine Verbindung **A1.2.** Das erfindungsgemäß eingesetzte Tri-Urethan-Triol **A1.1** ist erhältlich durch Umsetzung von cyclischen Carbonaten mit Verbindungen die mindestens drei Aminogruppen enthalten, wobei mindestens zwei der Aminogruppen primäre Aminogruppen sind. Beispielsweise sind die Tri-Urethan-Triole **A1.1** erhältlich durch Umsetzung von Propylencarbonat und/oder Ethylencarbonat mit Verbindungen die drei Aminogruppen enthalten, wobei mindestens zwei der Aminogruppen primäre Aminogruppen sind.

Die Tri-Urethan-Triole **A1.1** weisen eine Struktur gemäß der Formel (II) auf,

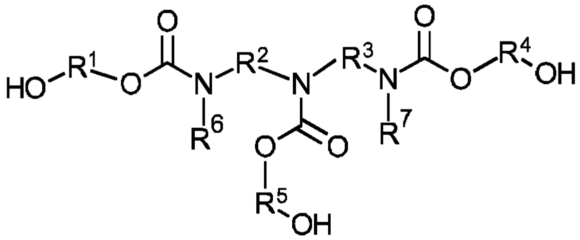

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^2$ bedeutet lineares oder verzweigtes $C_2$ bis $C_{24}$ - Alkylen, $C_3$ bis $C_{24}$ - Cycloalkylen, $C_4$ bis $C_{24}$-Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^3$ bedeutet lineares oder verzweigtes $C_2$ bis $C_{24}$ - Alkylen, $C_3$ bis $C_{24}$ - Cycloalkylen, $C_4$ bis $C_{24}$-Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können,

bevorzugt $C_2$ bis $C_{24}$ - Alkylen,

$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei $R^1$ bis $R^7$ identisch oder voneinander verschieden sein können.

Die Tri-Urethan-Triole **A1.1** gemäß der Formel (II) werden erhalten durch Umsetzung von cyclischen Carbonaten mit Triaminen gemäß Formel (III),

$$HN(R^6)\text{-}R^2\text{-}N(R^8)\text{-}R^3\text{-}NH(R^7) \qquad \text{(III)}$$

wobei $R^2$, $R^3$, $R^6$, $R^7$ und $R^8$ die oben genannte Bedeutung haben, wobei $R^2$, $R^3$, $R^6$, $R^7$ und $R^8$ identisch oder voneinander verschieden sein können.

Als cyclische Carbonate werden bevorzugt Propylencarbonat und/oder Ethylencarbonat eingesetzt.

Beispielsweise werden die Tri-Urethan-Triole **A1.1** gemäß der Formel (II) durch Umsetzung von Propylencarbonat und/oder Ethylencarbonat mit Triaminen gemäß Formel (III) erhalten.

Besonders bevorzugt werden die Tri-Urethan-Triole **A1.1** der Formel (II) durch Umsetzung von Propylencarbonat und/oder Ethylencarbonat mit mindestens einer Verbindung ausgewählt aus der Gruppe bestehend aus Diethylentriamin, 1,5,10-Triazadecan, 1,12-Diamino-4,9-diazadodekan, Dipropylentriamin, N-(2-Aminoethyl)-1,3-propandiamin und N,N'-Bis(3-aminopropyl)ethylendiamin erhalten.

Die Umsetzung der cyclischen Carbonate mit Verbindungen, die mindestens drei Aminogruppen enthalten, wobei mindestens zwei der Aminogruppen primäre Aminogruppen sind, erfolgt bevorzugt bei 50 bis 150 °C, besonders bevorzugt bei 80 bis 120 °C. Die Reaktionszeit liegt bevorzugt bei 4 bis 40 h, besonders bevorzugt bei 6 bis 30 h. Die Reaktion kann dabei in Anwesenheit eines Katalysators für die Aminolyse, wie z. B. 1,8-Diazabicyclo(5.4.0)undec-7-en, stattfinden.

In einer besonders vorteilhaften Ausführungsform wird das cyclische Carbonat mindestens äquimolar bezogen auf cyclisches Carbonat und Aminogruppen eingesetzt. Bevorzugt beträgt das molare Verhältnis von cyclischem Carbonat zu den Aminogruppen der Verbindungen die mindestens drei Aminogruppen enthalten, wobei mindestens zwei der Aminogruppen primäre Aminogruppen sind , 1,0 bis 4,0, besonders bevorzugt von 1,4 bis 3, ganz besonders bevorzugt von 2,0 bis 2,6.

Das überschüssige cyclische Carbonat kann direkt nach der Synthese des Tri-Urethan-Triols **A1.1** durch z.B. Dünnschichtverdampfung entfernt werden.

Es können jedoch auch molare Verhältnisse von cyclischem Carbonat zu den Aminogruppen der Verbindungen die mindestens drei Aminogruppen enthalten, wobei mindestens zwei der Aminogruppen primäre Aminogruppen sind, von unter 1,00 gewählt werden.

Der Anteil des Tri-Urethan-Triols **A1.1** in dem Verfahren kann 0,1 bis 35,0 Gew.-%, bevorzugt 3,0 bis 25,0 Gew.-%, besonders bevorzugt 5,0 bis 15,0 Gew.-% bezogen auf die Summe der Massen der Komponenten **A1.1** und **A1.2** betragen.

Es können in der Isocyanat-reaktiven Verbindung **A1** ein oder mehrere Tri-Urethan-Triole **A1.1** enthalten sein.

Neben dem Tri-Urethan-Triol **A1.1** werden im erfindungsgemäßen Verfahren mindestens eine Verbindung **A1.2** ausgewählt aus der Gruppe bestehend aus Polyetherpolyolen, Polyesterpolyolen, Polyetheresterpolyolen, Polycarbonatpolyolen und Polyethercarbonatpolyolen eingesetzt. Bevorzugt sind Polyesterpolyole und/oder Polyetherpolyole und ganz besonders bevorzugt Polyesterpolyole. Die Verbindung **A1.2** kann bevorzugt eine Hydroxylzahl zwischen 100 bis 550 mg KOH/g, insbesondere 100 bis 450 mg KOH/g und besonders bevorzugt 100 bis 350 mg KOH/g aufweisen. Vorzugsweise weist die einzelne Polyolkomponente ein zahlenmittleres Molekulargewicht von 120 g/mol bis 6000 g/mol, insbesondere 400 g/mol bis 2000 g/mol und besonders bevorzugt 500 g/mol bis 1000 g/mol auf. Die OH-Funktionalität der Verbindung **A1.2** kann 1,5 bis 4,0, bevorzugt 1,8 bis 3,0 und besonders bevorzugt 1,9 bis 2,5 betragen.

Die Polyesterpolyole der Verbindung **A1.2** können beispielsweise Polykondensate aus mehrwertigen Alkoholen, vorzugsweise Diolen, mit 2 bis 12 Kohlenstoffatomen, vorzugsweise mit 2 bis 6 Kohlenstoffatomen, und Polycarbonsäuren, wie z.B. Di-, Tri- oder sogar Tetracarbonsäuren, oder Hydroxycarbonsäuren oder Lactonen sein, bevorzugt werden aromatische Dicarbonsäuren oder Gemische aus aromatischen und aliphatischen Dicarbonsäuren verwendet. Es können aber auch nur eine oder mehrere aliphatische Polycarbonsäuren verwendet werden. Anstelle der freien Polycarbonsäuren können auch die entsprechenden Polycarbonsäureanhydride oder entsprechende Polycarbonsäureester von niederen Alkoholen zur Herstellung der Polyester verwendet werden, wobei bevorzugt Phthalsäureanhydrid eingesetzt wird.

Als Carbonsäuren kommen insbesondere in Betracht: Bernsteinsäure, Glutarsäure, Adipinsäure, Korksäure, Azelainsäure, Sebazinsäure, Decandicarbonsäure, Tetrahydrophthalsäure, Hexahydrophthalsäure, Cyclohexandicarbonsäure, Tetrachlorphthalsäure, Itaconsäure, Malonsäure, 2-Methylbernsteinsäure, 3,3-Diethylglutarsäure, 2,2-Dimethylbernsteinsäure, Dodekandisäure, Endomethylentetrahydrophthalsäure, Dimerfettsäure, Trimerfettsäure, Zitronensäure, Trimellithsäure, Benzoesäure, Trimellitsäure, Maleinsäure, Fumarsäure, Phthalsäure, Isophthalsäure und Terephthalsäure. Verwendet werden können ebenso Derivate dieser Carbonsäuren, wie beispielsweise Dimethylterephthalat. Die Carbonsäuren können dabei sowohl einzeln als auch im Gemisch verwendet werden. Als Carbonsäuren werden bevorzugt Terephthalsäure, Isophthalsäure, Adipinsäure, Sebacinsäure, Glutarsäure und/oder Bernsteinsäure, besonders bevorzugt Adipinsäure, Glutarsäure und/oder Bernsteinsäure und deren Gemische verwendet.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind unter anderem Caprolacton, Butyrolacton und Homologe.

Zur Herstellung der Polyesterpolyole kommen insbesondere auch biobasierte Ausgangsstoffe und/oder deren Derivate in Frage, wie z. B. Rizinusöl, Polyhydroxyfettsäuren, Ricinolsäure, Hydroxylmodifizierte Öle, Weintraubenkernöl, schwarzem Kümmelöl, Kürbiskernöl, Borretschsamenöl, Sojabohnenöl, Weizensamenöl, Rapsöl, Sonnenblumenkernöl, Erdnussöl, Aprikosenkernöl, Pistazienöl, Mandelöl, Olivenöl, Macadamianussöl, Avocadoöl, Sanddornöl, Sesamöl, Hanföl, Haselnussöl, Primelöl, Wildrosenöl, Distelöl, Walnussöl, Fettsäuren, hydroxylmodifizierte und epoxidierte Fettsäuren und Fettsäureester, beispielsweise basierend auf Myristoleinsäure, Palmitoleinsäure, Ölsäure, Vaccensäure, Petroselinsäure, Gadoleinsäure, Erukasäure, Nervonsäure, Linolsäure, alpha- und gamma-Linolensäure, Stearidonsäure, Arachidonsäure, Timnodonsäure, Clupanodonsäure und Cervonsäure. Insbesondere bevorzugt sind Ester der Rizinolsäure mit mehrfunktionellen Alkoholen, z.B. Glycerin. Bevorzugt ist auch die Verwendung von Mischungen solcher biobasierten Säuren mit anderen Carbonsäuren, z.B. Phthalsäuren.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester. Vorzugsweise verwendet werden Ethylenglykol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol. Ganz besonders bevorzugt werden Ethylenglykol und Diethylenglykol verwendet.

Daneben können auch Polyole wie Trimethylolpropan, Glycerin, Erythrit, Pentaerythrit, Trimethylolbenzol oder Trishydroxyethylisocyanurat eingesetzt werden, wobei Glycerin und Trimethylolpropan bevorzugt sind.

Es können zusätzlich auch einwertige Alkanole mit verwendet werden.

Erfindungsgemäß eingesetzte Polyetherpolyole werden nach dem Fachmann bekannten Herstellungsmethoden erhalten, wie beispielsweise durch anionische Polymerisation von einem oder mehreren Alkylenoxiden mit 2 bis 4 Kohlenstoffatomen mit Alkalihydroxiden, wie Natrium- oder Kaliumhydroxid, Alkalialkoholaten, wie Natriummethylat, Natrium- oder Kaliumethylat oder Kaliumisopropylat, oder aminischen Alkoxylierungs-Katalysatoren, wie Dimethylethanolamin (DMEOA), Imidazol und/oder Imidazolderivate, unter Verwendung mindestens eines Startermoleküls, das 2 bis 8, vorzugsweise 2 bis 6 reaktive Wasserstoffatome gebunden enthält.

Geeignete Alkylenoxide sind beispielsweise 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Bevorzugte Alkylenoxide sind Propylenoxid und Ethylenoxid.

Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 2,2'-, 2,4'- und 4,4'-Diaminodiphenylmethan.

Vorzugsweise verwendet werden zwei oder mehrwertige Alkohole, wie Ethandiol, 1,2- und 1,3-Propandiol, Diethylenglykol, Dipropylenglykol, 1,4-Butandiol, 1,6-Hexandiol, Triethanolamin, Bisphenole, Glycerin, Trimethylolpropan, Pentaerythrit, Sorbit und Saccharose.

Verwendbare Polycarbonatpolyole sind Hydroxylgruppen aufweisende Polycarbonate, zum Beispiel Polycarbonatdiole. Diese entstehen in der Reaktion von Kohlensäurederivaten, wie Diphenylcarbonat, Dimethylcarbonat oder Phosgen, mit Polyolen, bevorzugt Diolen.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenole und lactonmodifizierte Diole der vorstehend genannten Art.

Statt oder zusätzlich zu reinen Polycarbonatdiolen können auch Polyether-Polycarbonatdiole eingesetzt werden, welche beispielsweise durch Copolymerisation von Alkylenoxiden, wie zum Beispiel Propylenoxid, mit $CO_2$ erhältlich sind.

Verwendbare Polyetheresterpolyole sind solche Verbindungen, die Ethergruppen, Estergruppen und OH-Gruppen enthalten. Organische Dicarbonsäuren mit bis zu 12 Kohlenstoffatomen sind zur Herstellung der Polyetheresterpolyole geeignet, vorzugsweise aliphatische Dicarbonsäuren mit 4 bis 6 Kohlenstoffatomen oder aromatische Dicarbonsäuren, die einzeln oder im Gemisch verwendet werden.

Beispielhaft seien Korksäure, Azelainsäure, Decandicarbonsäure, Maleinsäure, Malonsäure, Phthalsäure, Pimelinsäure und Sebacinsäure sowie insbesondere Glutarsäure, Fumarsäure, Bernsteinsäure, Adipinsäure, Phthalsäure, Terephthalsäure und Isophthalsäure genannt. Neben organischen Dicarbonsäuren können auch Derivate dieser Säuren, beispielsweise deren Anhydride sowie deren Ester und Halbester mit niedermolekularen, monofunktionellen Alkoholen mit 1 bis 4 Kohlenstoffatomen eingesetzt werden. Der anteilige Einsatz der oben genannten biobasierten Ausgangsstoffe, insbesondere von Fettsäuren bzw. Fettsäurederivaten (Ölsäure, Sojaöl) ist ebenfalls möglich und kann Vorteile aufweisen, z.B. im Hinblick auf Lagerstabilität der Polyolformulierung, Dimensionsstabilität, Brandverhalten und Druckfestigkeit der Schäume.

Als weitere Komponente zur Herstellung der Polyetheresterpolyole werden Polyetherpolyole eingesetzt, die man durch Alkoxylieren von Startermolekülen wie mehrwertigen Alkoholen erhält. Die Startermoleküle sind mindestens difunktionell, können aber gegebenenfalls auch Anteile höherfunktioneller, insbesondere trifunktioneller, Startermoleküle enthalten.

Startermoleküle sind zum Beispiel Diole wie 1,2-Ethandiol, 1,3-Propandiol, 1,2-Propandiol, 1,4-Butandiol, 1,5-Pentendiol, 1,5-Pentandiol, Neopentylglykol, 1,6- Hexandiol, 1,7-Heptandiol, 1,8-Octandiol, 1,10-Decandiol, 2-Methyl-1,3-propandiol, 2,2-Dimethyl-1,3-propandiol, 3-Methyl-1,5-pentandiol, 2-Butyl-2-ethyl-1,3-propandiol, 2-Buten-1,4-diol und 2-Butin-1,4-diol, Etherdiole wie Diethylenglykol, Triethylenglykol, Tetraethylenglykol, Dibutylenglykol, Tributylenglykol, Tetrabutylenglykol, Dihexylenglykol, Trihexylenglykol, Tetrahexylenglykol und Oligomerengemische von Alkylenglykolen, wie Diethylenglykol. Auch Startermoleküle mit von OH verschiedenen Funktionalitäten können alleine oder in Mischung eingesetzt werden.

Neben den Diolen können als Startermoleküle für die Herstellung der Polyether auch Verbindungen mit mehr als 2 Zerewitinoff-aktiven Wasserstoffen, besonders mit zahlenmittleren Funktionalitäten von 3 bis 8, insbesondere von 3 bis 6 mitverwendet werden, zum Beispiel 1,1,1-Trimethylolpropan, Triethanolamin, Glycerin, Sorbitan und Pentaerythrit sowie auf Triolen oder Tetraolen gestartete Polyethylenoxidpolyole.

Polyetheresterpolyole können auch durch die Alkoxylierung, insbesondere durch Ethoxylierung und/oder Propoxylierung, von Reaktionsprodukten, die durch die Umsetzung von organischen Dicarbonsäuren und deren Derivaten sowie Komponenten mit Zerewitinoff-aktiven Wasserstoffen, insbesondere Diolen und Polyolen, erhalten werden, hergestellt werden. Als Derivate dieser Säuren können beispielsweise deren Anhydride eingesetzt werden, wie zum Beispiel Phthalsäureanhydrid. Herstellungsverfahren der Polyole sind beispielsweise von Ionescu in "Chemistry and Technology of Polyols for Polyurethanes", Rapra Technology Limited, Shawbury 2005, S.55 ff. (Kap. 4: Oligo-Polyols for Elastic Polyurethanes), S. 263 ff. (Kap. 8: Polyester Polyols for Elastic Polyurethanes) und insbesondere auf S.321 ff. (Kap. 13: Polyether Polyols for Rigid Polyurethane Foams) und S.419 ff. (Kap. 16: Polyester Polyols for Rigid Polyurethane Foams) beschrieben worden. Es ist auch möglich, Polyester- und Polyetherpolyole über Glykolyse von geeigneten Polymer-Rezyklaten zu gewinnen. Geeignete Polyether-Polycarbonatpolyole und ihre Herstellung werden beispielsweise in der EP 2 910 585 A1, [0024]-[0041], beschrieben. Beispiele zu Polycarbonatpolyolen und ihre Herstellung finden sich unter anderem in der EP 1 359 177 A1. Die Herstellung geeigneter Polyetheresterpolyole ist unter anderem in der WO 2010/043624 A und in der EP 1 923 417 A beschrieben.

Die Verbindung **A1.2** kann eine oder mehrere der oben genannten Verbindungen enthalten.

Weiterhin können in der Isocyanat-reaktiven Komponente **A1** niedermolekulare Isocyanat-reaktive Verbindungen **A1.3** enthalten sein, insbesondere können di- oder trifunktionelle Amine und Alkohole, besonders bevorzugt Diole und/oder Triole mit Molmassen $M_n$ kleiner als 400 g/mol, vorzugsweise von 60 bis 300 g/mol, zum Einsatz kommen, z.B. Triethanolamin, Diethylenglykol, Ethylenglykol und Glycerin. Sofern zur Herstellung der Polyurethan-Hartschaumstoffe solche niedermolekularen Isocyanat-reaktiven Verbindungen Anwendung finden, z.B. in der Funktion als Kettenverlängerungsmittel und/oder Vernetzungsmittel kommen diese zweckmäßigerweise in einer Menge von bis zu 5 Gew.-%, bezogen auf das Gesamtgewicht der Isocyanatreaktiven Komponente **A1**, zum Einsatz.

Neben den oben beschriebenen Polyolen und Isocyanatreaktiven Verbindungen können in der Isocyanatreaktiven Komponente **A1** weitere Isocyanat-reaktive Verbindungen **A1.4** enthalten sein, beispielsweise Graft-Polyole, Polyamine, Polyaminoalkohole und Polythiole. Selbstverständlich umfassen die beschriebenen Isocyanat-reaktiven Komponenten auch solche Verbindungen mit gemischten Funktionalitäten.

Die Isocyanat-reaktive Komponente **A1** kann eine oder mehrere der oben genannten Verbindungen enthalten. In einer bevorzugten Ausführungsform enthält die Isocyanatreaktive Komponente **A1** nur Tri-Urethan-Triol **A1.1** und Verbindung **A1.2.**

Als Flammschutzmittel **A2** können Verbindungen wie beispielsweise Phosphate oder Phosphonate, wie z. B. Diethylethylphosphonat (DEEP), Triethylphosphat (TEP), Triphenylphosphat (TPP), Trikresylphosphat, Diphenylkresylphosphat (DPK), Dimethylmethylphosphonat (DMMP), Diethanolaminomethylphosphonsäurediethylester, 9,10-Dihydro-9-oxa-10-phosphorylphenanthrene-10-oxid (DOPO) und Dimethylpropylphosphonat (DMPP), eingesetzt werden. Weitere geeignete Flammschutzmittel **A2** sind beispielsweise bromierte Ester, bromierte Ether (Ixol) oder bromierte Alkohole wie Dibromneopentylalkohol, Tribromneopentylalkohol, Tetrabromphthalat-Diol, sowie chlorierte Phosphate wie Tris(2-chlorethyl)phosphat, Tris-(2-chlorpropyl)phosphat (TCPP), Tris(1,3-dichlorpropyl)phosphat, Tris-(2,3-dibrompropyl)phosphat, Tetrakis-(2-chlorethyl)-ethylendiphosphat sowie handelsübliche halogenhaltige Flammschutzpolyole. Der Anteil des Flammschutzmittels **A2** kann 2,0 Gew.-% bis 30,0 Gew.-%, bevorzugt 4,0 Gew.-% bis 25,0 Gew.-%, besonders bevorzugt 8,0 Gew.-% bis 20,0 Gew.-%, bezogen auf die Summe der Massen der Komponenten **A1** bis **A5,** betragen.

Als Treibmittel **A3** kann physikalisches Treibmittel eingesetzt werden, wie beispielsweise niedrig siedende organische Verbindungen wie z. B. Kohlenwasserstoffe, halogenierte Kohlenwasserstoffe, Ether, Ketone, Carbonsäureester oder Kohlensäureester. Geeignet sind insbesondere organische Verbindungen, welche gegenüber der Isocyanatkomponente **B** inert sind und Siedepunkte unter 100 °C, vorzugsweise unter 50 °C bei Atmosphärendruck aufweisen. Diese Siedepunkte haben den Vorteil, dass die organischen Verbindungen unter dem Einfluss der exothermen Polyadditionsreaktion verdampfen. Beispiele solcher, vorzugsweise verwendeten organischen Verbindungen sind Alkane, wie Heptan, Hexan, n- und iso-Pentan, vorzugsweise technische Gemische aus n- und iso-Pentanen, n- und iso-Butan und Propan, Cycloalkane, wie z.B. Cyclopentan und/oder Cyclohexan, Ether, wie z. B. Furan, Dimethylether und Diethylether, Ketone, wie z. B. Aceton und Methylethylketon, Carbonsäurealkylester, wie z. B. Methylformiat, Dimethyloxalat und Ethylacetat und halogenierte Kohlenwasserstoffe, wie z. B. Methylenchlorid, Dichlormonofluormethan, Difluormethan, Trifluormethan, Difluorethan, Tetrafluorethan, Chlordifluorethane, 1,1-Dichlor-2,2,2-trifluorethan, 2,2-Dichlor-2-fluorethan und Heptafluorpropan. Auch bevorzugt ist der Einsatz von (hydro)fluorierten Olefinen, wie z. B. HFO 1233zd(E) (Trans-1-chlor-3,3,3-trifluor-1-propen) oder HFO 1336mzz(Z) (cis-1,1,1,4,4,4-Hexafluor-2-buten) oder Additive wie FA 188 von 3M (1,1,1,2,3,4,5,5,5-Nonafluor-4-(trifluormethyl)pent-2-en). Auch Gemische zweier oder mehrerer der genannten organischen Verbindungen können verwendet werden. Die organischen Verbindungen können dabei auch in Form einer Emulsion aus kleinen Tröpfchen eingesetzt werden.

Als Treibmittel A3 kann auch chemisches Treibmittel, wie beispielsweise Wasser, Carbonsäure und deren Gemische, verwendet werden. Diese reagieren mit Isocyanatgruppen unter Bildung des Treibgases, wie beispielsweise im Falle von Wasser entsteht dabei Kohlendioxid und im Falle von z. B. Ameisensäure entsteht dabei Kohlendioxid und Kohlenstoffmonoxid. Als Carbonsäure wird bevorzugt mindestens eine Verbindung, ausgewählt aus der Gruppe bestehend aus Ameisensäure, Essigsäure, Oxalsäure und Ricinolsäure, eingesetzt. Als chemisches Treibmittel wird besonders bevorzugt Wasser eingesetzt.

Vorzugsweise werden keine halogenierten Kohlenwasserstoffe als Treibmittel verwendet.

Als Treibmittel **A3** wird mindestens eine Verbindung, ausgewählt aus der Gruppe bestehend aus physikalischen und chemischen Treibmittel, eingesetzt. Bevorzugt wird nur physikalisches Treibmittel eingesetzt.

Als Katalysator **A4** zur Herstellung der PUR-/PIR-Hartschaumstoffe werden Verbindungen verwendet, welche die Reaktion der reaktiven Wasserstoffatome, insbesondere Hydroxylgruppen enthaltenden Verbindungen mit der Isocyanatkomponente **B** beschleunigen, wie z. B. tertiäre Amine oder Metallsalze. Die Katalysatorkomponenten können dem Reaktionsgemisch zudosiert oder auch ganz oder teilweise in der Isocyanatreaktiven Komponente **A1** vorgelegt werden.

Verwendet werden beispielsweise tertiäre Amine, wie Triethylamin, Tributylamin, Dimethylbenzylamin, Dicyclohexylmethylamin, Dimethylcyclohexylamin, N, N ,N', N'-Tetramethyldiaminodiethylether, Bis-(dimethylaminopropyl)-harnstoff, N-Methyl- bzw. N-Ethylmorpholin, N-Cyclohexylmorpholin, N,N,N',N'-Tetramethylethylendiamin, N,N,N,N-Tetramethylbutandiamin, N, N, N, N-Tetramethylhexandiamin-1,6, Pentamethyldiethylentriamin, Bis[2-(dimethylamino)ethyl]ether, Dimethylpiperazin, N-Dimethylaminoethylpiperidin, 1,2-Dimethylimidazol, 1-Azabicyclo-(3,3,0)-octan, 1,4-Diaza-bi-cyclo-(2,2,2)-octan (Dabco) und Alkanolaminverbindungen, wie Triethanolamin, Triisopropanolamin, N-Methyl- und N-Ethyldiethanolamin, Dimethylaminoethanol, 2-(N,N-Dimethylaminoethoxy)ethanol, N,N',N"-Tris-(dialkylaminoalkyl)hexahydrotriazin, z.B. N,N',N"-Tris-(dimethylaminopropyl)hexahydrotriazin und Triethylendiamin.

Es können auch Metallsalze, wie z. B. Alkali- oder Übergangsmetallsalze eingesetzt werden. Als Übergangsmetallsalze werden beispielsweise Zink-, Wismut-, Eisen-, Blei- oder bevorzugt Zinnsalze eingesetzt. Beispiele für eingesetzte Übergangsmetallsalze sind Eisen(II)-chlorid, Zinkchlorid, Bleioctoat, Zinndioctoat, Zinndiethylhexoat und Dibutylzinndilaurat. Besonders bevorzugt ist das Übergangsmetallsalz ausgewählt aus mindestens einer Verbindung aus der Gruppe bestehend aus Zinndioctoat, Zinndiethylhexoat und Dibutylzinndilaurat. Beispiele für Alkalimetallsalze sind Alkalialkoholate, wie z. B. Natriummethylat und Kaliumisopropylat, Alkalicarboxylate, wie z. B. Kaliumacetat, sowie Alkalimetallsalze von langkettigen Fettsäuren mit 10 bis 20 C-Atomen und gegebenenfalls seitenständigen OH-Gruppen. Bevorzugt als Alkalimetallsalz werden ein oder mehrere Alkalicarboxylate eingesetzt.

Als Katalysator **A4** kommen ferner in Betracht: Amidine, wie z. B. 2,3-Dimethyl-3,4,5,6-tetrahydropyrimidin, Tetraalkylammoniumhydroxide, wie z. B. Tetramethylammonium-hydroxid, Alkalihydroxide, wie z. B. Natriumhydroxid, und Tetraalkylammonium- oder Phosphoniumcarboxylate. Darüber hinaus sind Mannichbasen und Salze von Phenolen geeignete Katalysatoren.

Wird beim Verschäumen ein größerer Polyisocyanatüberschuss verwendet, kommen als Katalysatoren für die Trimerisierungsreaktion der überschüssigen NCO-Gruppen untereinander ferner in Betracht: Isocyanuratgruppen ausbildende Katalysatoren, beispielsweise Ammoniumionen- oder Alkalimetallsalze, speziell Ammonium- oder Alkalimetallcarboxylate, alleine oder in Kombination mit tertiären Aminen. Die Isocyanurat-Bildung führt zu besonders flammwidrigen PIR-Schaumstoffen.

Die oben genannten Katalysatoren können alleine oder in Kombination miteinander eingesetzt werden. Gegebenenfalls können ein oder mehrere Hilfs- und Zusatzstoffe als Komponente **A5** eingesetzt werden. Beispiele für die Komponente **A5** sind oberflächenaktive Substanzen, Zellregler, Füllstoffe, Farbstoffe, Pigmente, Hydrolyseschutzmittel, fungistatische und bakteriostatisch wirkende Substanzen.

Als oberflächenaktive Substanzen kommen z.B. Verbindungen in Betracht, welche zur Unterstützung der Homogenisierung der Ausgangsstoffe dienen und gegebenenfalls auch geeignet sind, die Zellstruktur der Kunststoffe zu regulieren. Genannt seien beispielsweise Emulgatoren, wie die Natriumsalze von Ricinusölsulfaten oder von Fettsäuren sowie Salze von Fettsäuren mit Aminen, z.B. ölsaures Diethylamin, stearinsaures Diethanolamin, ricinolsaures Diethanolamin, Salze von Sulfonsäuren, z.B. Alkali- oder Ammoniumsalze von Dodecylbenzol- oder Dinaphthylmethandisulfonsäure und Ricinolsäure; Schaumstabilisatoren, wie Siloxanoxalkylen-Mischpolymerisate und andere Organopolysiloxane, oxethylierte Alkylphenole, oxethylierte Fettalkohole, Paraffinöle, Rizinusöl- bzw. Ricinolsäureester, Türkischrotöl und Erdnussöl, und Zellregler, wie Paraffine, Fettalkohole und Dimethylpolysiloxane.

Als Füllstoffe, insbesondere verstärkend wirkende Füllstoffe, sind die an sich bekannten, üblichen organischen und anorganischen Füllstoffe, Verstärkungsmittel, Beschwerungsmittel, Mittel zur Verbesserung des Abriebverhaltens in Anstrichfarben, Beschichtungsmittel usw. zu nennen. Im Einzelnen seien beispielhaft genannt: anorganische Füllstoffe wie silikatische Mineralien, beispielsweise Schichtsilikate wie z. B. Antigorit, Serpentin, Hornblenden, Amphibole, Chrisotil, Montmorillonit und Talkum, Metalloxide, wie Kaolin, Aluminiumoxide, Titanoxide und Eisenoxide, Metallsalze, wie Kreide, Schwerspat und anorganische Pigmente, wie Cadmiumsulfid und Zinksulfid, sowie Glas u.a. sowie natürliche und synthetische faserförmige Mineralien wie Wollastonit, Metall- und insbesondere Glasfasern verschiedener Länge, die gegebenenfalls geschlichtet sein können. Als organische Füllstoffe kommen beispielsweise in Betracht: Kohle, Melamin, Kolophonium, Cyclopentadienylharze und Pfropfpolymerisate sowie Cellulosefasern, Polyamid-, Polyacrylnitril-, Polyurethan-, Polyesterfasern auf der Grundlage von aromatischen und/oder aliphatischen Dicarbonsäureestern und Kohlenstofffasern.

Als geeignete organische Polyisocyanatkomponente **B** sind aliphatische, cycloaliphatische, araliphatische, aromatische und heterocyclische Polyisocyanate, wie sie z.B. von W. Siefken in Justus Liebigs Annalen der Chemie, 562, Seiten 75 bis 136 beschrieben werden, beispielsweise solche der Formel (IV),

$$Q(NCO)_n , \qquad (IV)$$

in der

n = 2 - 4, vorzugsweise 2 - 3,
und
Q einen aliphatischen Kohlenwasserstoffrest mit 2 - 18, vorzugsweise 6 - 10 C-Atomen, einen cycloaliphatischen Kohlenwasserstoffrest mit 4 - 15, vorzugsweise 6 - 13 C-Atomen oder einen araliphatischen Kohlenwasserstoffrest mit 8 - 15, vorzugsweise 8 - 13 C-Atomen bedeuten.

Beispielsweise handelt es sich um solche Polyisocyanate, wie sie in der EP 0 007 502 A1, Seiten 7 - 8, beschrieben werden. Bevorzugt werden in der Regel die technisch leicht zugänglichen Polyisocyanate, z.B. das 2,4- und 2,6-Toluylendiisocyanat, sowie beliebige Gemische dieser Isomeren ("TDI"); Polyphenylpolymethylenpolyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten. Die urethangruppenhaltigen Polyisocyanate (Präpolymere) können beispielsweise Reaktionsprodukte der Polyisocyanate mit Polyester-Polyolen oder aber beliebigen anderen Polyolen (beispielsweise konventionellen Polyetherpolyolen) sein. Vorzugsweise wird als Polyisocyanat mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt, besonders bevorzugt wird als Polyisocyanat eine Mischung enthaltend 4,4'-Diphenylmethandiisocyanat und 2,4'-Diphenylmethandiisocyanat und Polyphenylpolymethylenpolyisocyanat eingesetzt.

Es ist möglich, dass im Reaktionsgemisch die Anzahl der NCO-Gruppen im Isocyanat und die Anzahl der gegenüber Isocyanaten reaktiven Gruppen zu einer Kennzahl (Index) von 90 bis 600 führen, bevorzugt zwischen 115 und 400. Diese Kennzahl liegt bevorzugt in einem Bereich von 150 bis 450 in dem ein hoher Anteil an Polyisocyanuraten (PIR) vorliegt (der Hartschaumstoff wird als PIR-Hartschaumstoff oder PUR-/PIR-Hartschaumstoff bezeichnet) und zu einer höheren Flammwidrigkeit des PUR-/PIR-Hartschaumstoffes selbst führt. Ein anderer bevorzugter Bereich für die Isocyanat-Kennzahl ist der Wertebereich von 90 bis 150 (der Hartschaumstoff wird als Polyurethanhartschaumstoff (PUR-Hartschaumstoff) bezeichnet) mit zum Beispiel einer geringeren Sprödigkeit im Vergleich zu PUR-/PIR-Hartschaumstoff.

Unter der Isocyanat-Kennzahl (auch Kennzahl oder Isocyanat-Index genannt) wird der Quotient aus der tatsächlich eingesetzten Stoffmenge [Mol] an Isocyanat-Gruppen und der tatsächlich eingesetzten Stoffmenge [Mol] an Isocyanat-reaktiven Gruppen, multipliziert mit 100, verstanden:

$$\text{Kennzahl} = (\text{Mole Isocyanat-Gruppen} / \text{Mole Isocyanat-reaktive Gruppen}) * 100$$

Der NCO-Wert (auch: NCO-Gehalt, Isocyanatgehalt) wird bestimmt mittels EN ISO 11909 (Mai 2007).

Die Erfindung betrifft ebenfalls einen PUR-/PIR-Hartschaumstoff, der durch das erfindungsgemäße Verfahren hergestellt ist.

Die Herstellung der erfindungsgemäßen PUR-/PIR-Hartschaumstoffe erfolgt bevorzugt nach dem Fachmann bekannten Einstufenverfahren, bei dem die Reaktionskomponenten kontinuierlich oder diskontinuierlich miteinander zur Reaktion gebracht werden und dann anschließend entweder manuell oder durch Zuhilfenahme maschineller Einrichtungen im Hochdruck- oder Niederdruckverfahren nach Austrag auf ein Transportband oder in geeignete Formen zur Aushärtung gebracht werden. Beispiele sind in US-A 2 764 565, in G. Oertel (Hrsg.) "Kunststoff-Handbuch", Band VII, Carl Hanser Verlag, 3. Auflage, München 1993, S. 267 ff., sowie in K. Uhlig (Hrsg.) "Polyurethan Taschenbuch", Carl Hanser Verlag, 2. Auflage, Wien 2001, S. 83-102 beschrieben. Die PUR-/PIR-Hartschaumstoffe können eine Rohdichte von 25,0 bis 300,0 kg/$m^3$, bevorzugt 25,0 bis 80,0 kg/$m^3$, besonders bevorzugt 30,0 bis 65,0 kg/$m^3$ und insbesondere 30,0 bis 45,0 kg/$m^3$ aufweisen.

Die erfindungsgemäßen PUR-/PIR-Hartschaumstoffe finden bevorzugt zur Herstellung von Verbundelementen Verwendung. Üblicherweise findet hier die Verschäumung in kontinuierlicher oder diskontinuierlicher Weise gegen mindestens eine Deckschicht statt.

Ein weiterer Gegenstand der Erfindung ist demzufolge die Verwendung eines erfindungsgemäßen PUR-/PIR-Hartschaumstoffes als Isolationsschaum und/oder als Haftvermittler in Verbundelementen, wobei die Verbundelemente eine einen erfindungsgemäßen PUR-/PIR-Hartschaumstoff umfassende Schicht und mindestens eine Deckschicht umfassen. Die Deckschicht wird hierbei zumindest teilweise von einer den erfindungsgemäßen PUR-/PIR-Hartschaumstoff umfassenden Schicht kontaktiert. Verbundelemente der hier interessierenden Art werden auch als Sandwich-Elemente oder Dämmplatten bezeichnet und dienen in der Regel als Bauelemente für den Schallschutz, die thermische Dämmung, zum Hallenbau oder für den Fassadenbau. Es kann sich bei den einen oder zwei Deckschichten jeweils um eine flexible Deckschicht, z.B. um eine Aluminium-Folie, Papier, Kunststoffbahnen, Multischicht-Deckschichten aus Papier und Aluminium oder aus Mineralvließ, und/oder um eine starre Deckschicht, z.B. aus Stahlblech oder bis zu 7 mm starke Spanplatten, handeln, abhängig vom Einsatzzweck der Verbundelemente.

**Beispiele:**

**Messmethoden:**

| | |
|---|---|
| Hydroxylzahl: | Die Bestimmung der OH-Zahl erfolgte gemäß der Vorschrift der DIN 53240-1 (Verfahren ohne Katalysator, Fassung v. Juni 2013). |
| Aminzahl: | Gesamtbasenzahl gemäß DIN 51639-1 in der Fassung von Nov. 2014. |
| Säurezahl: | Die Säurezahl wurde gemäß DIN EN ISO 2114 (Juni 2002) bestimmt. |
| Abbindezeit: | Die Abbindezeit ("Gelpunkt $t_G$") wird ermittelt, indem man ein Holzstäbchen in die reagierende Polymerschmelze eintaucht und wieder herausnimmt. Sie charakterisiert den Zeitpunkt, ab dem sich die Polymerschmelze verhärtet. Als $t_G$ wird der Zeitpunkt angegeben, an dem sich erstmalig Fäden zwischen Holzstab und Polymerschmelze ziehen lassen. |
| Startzeit: | Die Zeitspanne, die vom Start der Vermischung der Hauptkomponenten bis zum sichtbaren Beginn der Schäumung des Gemisches verstreicht. |
| Viskosität: | Es wurde die dynamische Viskosität mit dem Rheometer MCR 51 der Firma Anton Paar entsprechend DIN 53019-1 (Fassung v. September 2008) mit einem Messkegel CP 50-1 (Durchmesser 50 mm, Winkel 1°) bei Scherraten von 25 $s^{-1}$, 100 $s^{-1}$, 200 $s^{-1}$ und 500 $s^{-1}$ gemessen. Die erfindungsgemäßen und nicht erfindungsgemäßen Polyole zeigen von der Scherrate unabhängige Viskositätswerte. |
| Rohdichte: | Die Rohdichte wurde gemäß DIN EN ISO 845 (Fassung vom Oktober 2009) ermittelt. |
| Brandeigenschaften: | Die Brandeigenschaften wurden gemäß DIN 4102-1 (Fassung v. Mai 1998) bestimmt. |
| Stauchhärte: | Die Stauchhärte der PUR-/PIR-Hartschaumstoffe wurden gemäß DIN EN ISO 844 (Fassung vom November 2014) bestimmt. |
| Offenzelligkeit: | Die Offenzelligkeit der PUR-/PIR-Hartschaumstoffe wurde nach DIN EN ISO 4590 (Fassung v. Juni 2014) ermittelt. |

**Eingesetzte Rohstoffe:**

| | |
|---|---|
| cPC: | Cyclisches Propylencarbonat (Fa. Acros) |
| DBU: | 1,8-Diazabicyclo(5.4.0)undec-7-en |
| DETA: | Diethylentriamin |
| A1.2-1: | Aliphatisches Polyesterpolyol aus technischer Glutarsäure (Gemisch aus Glutarsäure, Bernsteinsäure und Adipinsäure, Fa. Lanxess AG) und Ethylenglykol, Hydroxylzahl: 237 mg KOH/g, Säurezahl: 1,9 mg KOH/g, Viskosität: 1160 mPas bei 25°C |
| A1.2-2: | Lineares Polyesterpolyol, Hydroxylzahl: 240 mg KOH/g (Desmophen®2382, Fa. Covestro Deutschland GmbH) |
| A1.2-3: | Enthaltend das Umsetzungsprodukt von Phthalsäureanhydrid und Diethylenglykol, Säurezahl: 97 mg KOH/g (Additiv 1132, Fa. Covestro Deutschland AG) |
| A1.2-4: | Polyetherpolyol auf Aminbasis, Hydroxylzahl: 395 bis 435 mg KOH/g, Viskosität: 6450 - 9550 mPas bei 25°C (Desmophen®T 460, Fa. Covestro Deutschland GmbH) |
| A2-1: | Trischlorisopropylphosphat (Levagard®PP, Fa. Lanxess AG) |
| A2-2: | Triethylphosphat (Levagard®TEP-Z, Fa. Lanxess AG) |
| A3-1: | n-Pentan (Fa. Kraemer&Martin GmbH) |
| A4-1: | Aktivator für die Herstellung von Polyurethan-Hartschaumstoffen (Desmorapid®DB, Fa. Covestro Deutschland AG) |
| A4-2: | Kaliumacetat in Diethylenglykol (Desmorapid®PU 1792, Fa. Covestro Deutschland AG) |
| A5-1: | Modifiziertes Polyethersiloxan (Tegostab®B 8443, Fa. Evonik) |
| B-1: | Polyisocyanat mit einem NCO-Gehalt von 30,5 bis 32,5 Gew.-% (Desmodur®44V20 L, Fa. Covestro Deutschland AG) |

**Herstellung von Tri-Urethan-Triol A1.1-1:**

In einem 500 mL Planschliffreaktor mit beheizbarem Doppelmantel und elektrischem Rührer werden 348,7 g (3,42 mol) cPC und 2,22 g (14,6 mmol) DBU unter Inertgas ($N_2$) vorgelegt. Danach wird im Verlauf einer Stunde unter Rühren und 100 °C 50,3 g (0,49 mol) DETA zugetropft. Im Anschluss wurde die Reaktion für insgesamt 6 h bei 100 °C

nachgerührt.

Überschüssiges cPC wurde durch Kurzwegverdampfung bei einem Druck von 0,01 mbar entfernt, wobei die Manteltemperatur 140 °C betrug und eine Dosiergeschwindigkeit von 200 g/Stunde gewählt wurde.

Analyse:

| | |
|---|---|
| Viskosität: | 95500 mPas (50 °C), 3900 mPas (75 °C) |
| Hydroxylzahl: | 366 mg KOH/g |
| Aminzahl: | 43,3 mg KOH/g |

**Herstellung einer Mischung enthaltend ein Tri-Urethan-Triol A1.1 und eine Verbindung A1.2:**

10 g des Tri-Urethan-Triols A1.1-2 werden in einem Trockenschrank auf 100°C vorgewärmt und zu 90 g auf 60°C vorgewärmtes A1.2-1 zugegeben. Die Mischung wird mittels eines Pendraulikrührers bis zur Homogenität verrührt und besitzt eine Hydroxylzahl von 250 mg KOH/g.

**Herstellung der PUR-/PIR-Hartschaumstoffe:**

Zur Herstellung der PUR-/PIR-Hartschaumstoffe werden die Isocyanatreaktiven Komponenten, Flammschutzmittel, Katalysatoren, Treibmittel und Schaumstabilisator vermischt, zu der erhaltene Mischung Polyisocyanat zugesetzt und das Gemisch in eine Papierform (30x30x10 $cm^3$) gegossen und darin ausreagiert. Die Rezepturen und Ergebnisse der physikalischen Messungen an den erhaltenen Proben sind in der Tabelle 1 wiedergegeben.

Tabelle 1: Herstellung von PUR-/PIR-Hartschaumstoffen unter Verwendung von A1.1-1

| **Beispiel** | | **1 (Vgl.)** | **2** | **3** | **4** |
|---|---|---|---|---|---|
| A1.2-2 | [g] | 74,9 | | | |
| Mischung enthaltend TUT A1.1-1 und Polyesterpolyol A1.2-1 | [g] | | 76,4 | 79,2 | 80,7 |
| A1.2-3 | [g] | 2,59 | 2,64 | 2,74 | 2,79 |
| A1.2-4 | [g] | 5,86 | 5,97 | 6,19 | 6,31 |
| A2-1 | [g] | 23,4 | 23,9 | 12,4 | 6,3 |
| A2-2 | [g] | 5,86 | 5,97 | 6,19 | 6,31 |
| A3-1 | [g] | 16,13 | 15,92 | 15,82 | 15,77 |
| A4-1 | [g] | 1,69 | 2,87 | 2,98 | 3,03 |
| A4-2 | [g] | 4,17 | 3,22 | 3,34 | 3,4 |
| A5-1 | [g] | 3,94 | 4,02 | 4,17 | 4,25 |
| B-1 | [g] | 211,4 | 209,1 | 217 | 221,1 |
| Isocyanat -Kennzahl | | 325,1 | 325,1 | 325,1 | 325,1 |

**Tabelle 1 (Fortsetzung):** Analyse

| **Beispiel** | | **1 (Vgl.)** | **2** | **3** | **4** |
|---|---|---|---|---|---|
| **Verarbeitung:** | | | | | |
| Startzeit | [s] | 15 | 17 | 17 | 18 |
| Gelzeit | [s] | 43 | 47 | 46 | 46 |
| Klebfreizeit | [s] | 63 | 65 | 61 | 66 |

(fortgesetzt)

| **Eigenschaften:** | | | | | |
|---|---|---|---|---|---|
| Rohdichte, Kern | [kg/m$^3$] | 42,3 | 41,6 | 41,4 | 41,2 |
| Stauchhärte, quer | [kPa] | 225 | 202 | 205 | 209 |
| Stauchhärte, parallel | [kPa] | 375 | 318 | 336 | 342 |
| Offenzelligkeit | [%] | 5,2 | 5,7 | 5,7 | 5,1 |
| Flammhöhe | [mm] | 140-145 | 125-130 | 135-140 | 130-140 |
| Klassifizierung | | B2 | B2 | B2 | B2 |

Die Tabelle 1 zeigt in den Beispielen 2 bis 4, dass die Verwendung der erfindungsgemäßen Mischung zu PUR-/PIR-Hartschaumstoffen mit einer Brandschutzklasse B2 führt. Verglichen mit dem Vergleichsbeispiel 1 besitzen die PUR-/PIR-Hartschaumstoffe nach dem erfindungsgemäßen Verfahren (erfindungsgemäße Beispiele 2 bis 4) einen verbesserten Flammschutz (siehe Tabelle 1: Analyse). Die erfindungsgemäßen Beispiele 3 und 4 zeigen, dass der Anteil an Flammschutzmittel in den PUR-/PIR-Hartschaumstoffen, insbesondere des halogenhaltigen Flammschutzmittels TCPP, verringert werden kann. Der Flammschutz der PUR-/PIR-Hartschaumstoffen ist trotz eines verringerten Anteils an Flammschutzmittel mindestens gleichwertig zum Flammschutz der PUR-/PIR-Hartschaumstoffe des Vergleichsbeispiels 1.

Weiterhin belegt die Tabelle 1 (Analyse), dass alle verarbeitungstechnischen relevanten Größen, wie z.B. Startzeit, Gelzeit und Klebfreizeit gegenüber dem Vergleichsbeispiel 1 praktisch unverändert erhalten bleiben, wenn kleinere Anpassungen der Katalysatoren und Stabilisatoren erfolgen. Unter diesen Voraussetzungen behalten auch alle mechanischen Eigenschaften der PUR-/PIR-Hartschaumstoffe der erfindungsgemäßen Beispiele das vom Vergleichsbeispiel 1 vorgegebene Niveau bei.

## Patentansprüche

**1.** Verfahren zur Herstellung von PUR-/PIR-Hartschaumstoffen, enthaltend

**A1** eine Isocyanatreaktive Komponente
**A2** Flammschutzmittel
**A3** Treibmittel
**A4** Katalysator
**A5** gegebenenfalls Hilfs- und Zusatzstoffe
**B** eine organische Polyisocyanatkomponente
**dadurch gekennzeichnet, dass**
die Komponente **A1** ein Tri-Urethan-Triol **A1.1** und eine Verbindung **A1.2** ausgewählt aus der Gruppe bestehend aus Polyetherpolyol, Polyesterpolyol, Polyethercarbonatpolyol und Polyetheresterpolyol enthält, wobei das Tri-Urethan-Triol **A1.1** eine Struktur gemäß der Formel (II) aufweist,

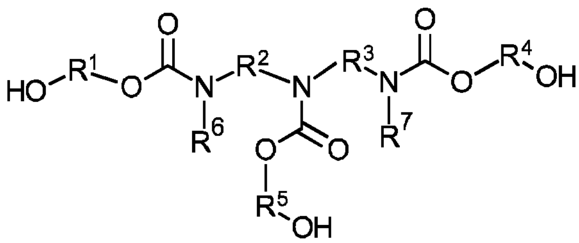

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^2$ bedeutet lineares oder verzweigtes $C_2$ bis $C_{24}$ - Alkylen, $C_3$ bis $C_{24}$ - Cycloalkylen, $C_4$ bis $C_{24}$ - Arylen,

$C_5$ bis $C_{24}$ - Aralkylen, $C_2$ bis $C_{24}$ - Alkenylen, $C_2$ bis $C_{24}$-Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^3$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$-Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei $R^1$ bis $R^7$ identisch oder voneinander verschieden sein können.

2. Verfahren gemäß Anspruch 1, **dadurch gekennzeichnet, dass** die Isocyanat-reaktive Komponente **A1** eine Verbindung **A1.2** mit einer Hydroxylzahl von 100 bis 550 mg KOH/g, bevorzugt 100 bis 450 mg KOH/g, besonders bevorzugt 100 bis 350 mg KOH/g enthält.

3. Verfahren gemäß Anspruch 1 oder 2, **dadurch gekennzeichnet, dass** die Isocyanat-reaktive Komponente **A1** eine Verbindung **A1.2** mit einer OH-Funktionalität von 1,5 bis 4,0, bevorzugt 1,8 bis 3,0, besonders bevorzugt 1,9 bis 2,5 enthält.

4. Verfahren gemäß einem der Ansprüche 1 bis 3, **dadurch gekennzeichnet, dass** die Verbindung **A1.2** ein Polyesterpolyol oder ein Polyetherpolyol, bevorzugt ein Polyesterpolyol ist.

5. Verfahren gemäß einem der Ansprüche 1 bis 4, **dadurch gekennzeichnet, dass** die Verbindung **A1.2** ein Polyesterpolyol erhältlich aus der Reaktion aliphatischer und/oder aromatischer Dicarbonsäure mit mindestens einem aliphatischen Diol ist.

6. Verfahren gemäß einem der Ansprüche 1 bis 5, **dadurch gekennzeichnet, dass** das Tri-Urethan-Triol **A1.1** erhalten wird aus der Reaktion zwischen cyclischen Ethylencarbonat und/oder cyclischen Propylencarbonat mit einer Verbindung enthaltend mindestens drei Aminogruppen, wobei mindestens zwei der Aminogruppen primäre Aminogruppen sind.

7. Verfahren gemäß einem der Ansprüche 1 bis 6, **dadurch gekennzeichnet, dass** der Anteil an Tri-Urethan-Triol **A1.1** 0,1 bis 35,0 Gew.-%, bevorzugt 3,0 bis 25,0 Gew.-%, besonders bevorzugt 5,0 bis 15,0 Gew.-%, bezogen auf die Summe der Massen der Komponenten **A1.1** und **A1.2** , beträgt.

8. Verfahren gemäß einem der Ansprüche 1 bis 7, **dadurch gekennzeichnet, dass** die Isocyanat-Kennzahl 100 bis 500, bevorzugt 180 bis 450 beträgt.

9. Verfahren gemäß einem der Ansprüche 1 bis 8, **dadurch gekennzeichnet, dass** das Flammschutzmittel **A2** in einem Anteil von 2,0 Gew.-% bis 30,0 Gew.-%, bevorzugt 4,0 Gew.-% bis 25,0 Gew.-%, besonders bevorzugt 8,0 Gew.-% bis 20,0 Gew.-%, bezogen auf das Gewicht der Komponenten **A1** bis **A5.**

10. Verfahren gemäß einem der Ansprüche 1 bis 9, **dadurch gekennzeichnet, dass** als Polyisocyanatkomponente **B** mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt wird.

**11.** PUR-/PIR-Hartschaumstoff erhältlich durch ein Verfahren gemäß einem der Ansprüche 1 bis 10.

**12.** PUR-/PIR-Hartschaumstoff gemäß Anspruch 11, **dadurch gekennzeichnet, dass** die Rohdichte 25,0 bis 300,0 $kg/m^3$, bevorzugt 25,0 bis 80,0 $kg/m^3$, besonders bevorzugt 30,0 bis 65,0 $kg/m^3$ und insbesondere 30,0 bis 45,0 $kg/m^3$ beträgt.

**13.** Verwendung eines PUR-/PIR-Hartschaumstoffes gemäß einem der Ansprüche 11 oder 12 als Dämmmaterial oder Verbundelement mit flexiblen oder nicht-flexiblen Deckschichten.

**14.** Mischung enthaltend ein Tri-Urethan-Triol **A1.1** und eine Verbindung **A1.2, dadurch gekennzeichnet, dass** die Verbindung **A1.2** ausgewählt ist aus der Gruppe bestehend aus Polyetherpolyol, Polyesterpolyol, Polyethercarbonatpolyol und Polyetheresterpolyol, wobei das Tri-Urethan-Triol **A1.1** eine Struktur gemäß der Formel (II) aufweist,

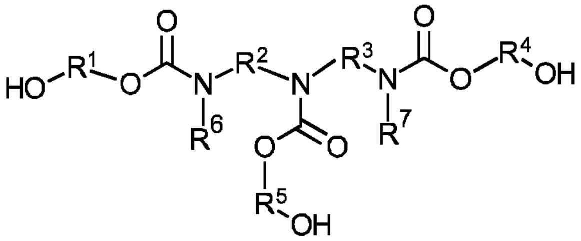

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^2$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$-Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,
$R^3$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$-Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,
$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,
$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei $R^1$ bis $R^7$ identisch oder voneinander verschieden sein können.

## Claims

**1.** Process for producing rigid PUR/PIR foams containing

**A1** an isocyanate-reactive component
**A2** flame retardant
**A3** blowing agent

**A4** catalyst
**A5** optionally auxiliaries and additives
**B** an organic polyisocyanate component
**characterized in that**
component **A1** contains a triurethane triol **A1.1** and a compound **A1.2** selected from the group consisting of polyether polyol, polyester polyol, polyether carbonate polyol and polyether ester polyol, wherein the triurethane triol **A1.1** has a structure of formula (II)

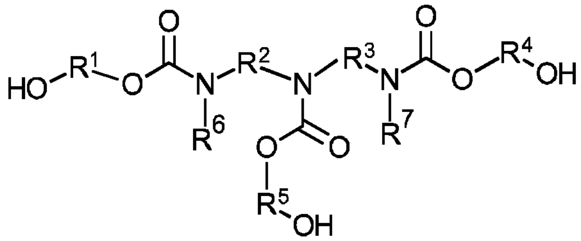

(II)

where

$R^1$ is linear or branched $C_2$- to $C_{24}$-alkylene which may optionally be interrupted by heteroatoms such as O, S or N and may be substituted, preferably $CH_2$-$CH_2$ or $CH_2$-$CH(CH_3)$,
$R^2$ is linear or branched $C_2$- to $C_{24}$-alkylene, $C_3$- to $C_{24}$-cycloalkylene, $C_4$- to $C_{24}$-arylene, $C_5$- to $C_{24}$-aralkylene, $C_2$- to $C_{24}$-alkenylene, $C_2$- to $C_{24}$-alkynylene, each of which may optionally be interrupted by heteroatoms such as O, S or N and/or each of which may be substituted by alkyl, aryl and/or hydroxyl, preferably $C_2$- to $C_{24}$-alkylene,
$R^3$ is linear or branched $C_2$- to $C_{24}$-alkylene, $C_3$- to $C_{24}$-cycloalkylene, $C_4$- to $C_{24}$-arylene, $C_5$- to $C_{24}$-aralkylene, $C_2$- to $C_{24}$-alkenylene, $C_2$- to $C_{24}$-alkynylene, each of which may optionally be interrupted by heteroatoms such as O, S or N and/or each of which may be substituted by alkyl, aryl and/or hydroxyl, preferably $C_2$- to $C_{24}$-alkylene,
$R^4$ is linear or branched $C_2$- to $C_{24}$-alkylene which may optionally be interrupted by heteroatoms such as O, S or N and may be substituted, preferably $CH_2$-$CH_2$ or $CH_2$-$CH(CH_3)$,
$R^5$ is linear or branched $C_2$- to $C_{24}$-alkylene which may optionally be interrupted by heteroatoms such as O, S or N and may be substituted, preferably $CH_2$-$CH_2$ or $CH_2$-$CH(CH_3)$,
$R^6$ is H, linear or branched $C_1$- to $C_{24}$-alkyl, $C_3$- to $C_{24}$-cycloalkyl, $C_4$- to $C_{24}$-aryl, $C_5$- to $C_{24}$-aralkyl, $C_2$- to $C_{24}$-alkenyl, $C_2$- to $C_{24}$-alkynyl, each of which may optionally be interrupted by heteroatoms such as O, S or N and/or each of which may be substituted by alkyl, aryl and/or hydroxyl, preferably H,
$R^7$ is H, linear or branched $C_1$- to $C_{24}$-alkyl, $C_3$- to $C_{24}$-cycloalkyl, $C_4$- to $C_{24}$-aryl, $C_5$- to $C_{24}$-aralkyl, $C_2$- to $C_{24}$-alkenyl, $C_2$- to $C_{24}$-alkynyl, each of which may optionally be interrupted by heteroatoms such as O, S or N and/or each of which may be substituted by alkyl, aryl and/or hydroxyl, preferably H,

and wherein $R^1$ to $R^7$ may be identical or different from one another.

2. Process according to Claim 1, **characterized in that** the isocyanate-reactive component **A1** contains a compound **A1.2** having a hydroxyl number of 100 to 550 mg KOH/g, preferably 100 to 450 mg KOH/g, particularly preferably 100 to 350 mg KOH/g.

3. Process according to Claim 1 or 2, **characterized in that** the isocyanate-reactive component **A1** contains a compound **A1.2** having an OH functionality of 1.5 to 4.0, preferably 1.8 to 3.0, particularly preferably 1.9 to 2.5.

4. Process according to any of Claims 1 to 3, **characterized in that** compound **A1.2** is a polyester polyol or a polyether polyol, preferably a polyester polyol.

5. Process according to any of Claims 1 to 4, **characterized in that** compound **A1.2** is a polyester polyol obtainable from the reaction of aliphatic and/or aromatic dicarboxylic acid with at least one aliphatic diol.

6. Process according to any of Claims 1 to 5, **characterized in that** the triurethane triol **A1.1** is obtained from the reaction between cyclic ethylene carbonate and/or cyclic propylene carbonate with a compound containing at least

three amino groups, where at least two of the amino groups are primary amino groups.

7. Process according to any of Claims 1 to 6, **characterized in that** the proportion of triurethane triol **A1.1** is 0.1% to 35.0% by weight, preferably 3.0% to 25.0% by weight, particularly preferably 5.0% to 15.0% by weight, based on the sum total of the masses of components **A1.1** and **A1.2.**

8. Process according to any of Claims 1 to 7, **characterized in that** the isocyanate index is 100 to 500, preferably 180 to 450.

9. Process according to any of Claims 1 to 8, **characterized in that** flame retardant **A2** in a proportion of 2.0% by weight to 30.0% by weight, preferably 4.0% by weight to 25.0% by weight, particularly preferably 8.0% by weight to 20.0% by weight, based on the weight of components **A1** to **A5.**

10. Process according to any of Claims 1 to 9, **characterized in that** the polyisocyanate component **B** used is at least one compound selected from the group consisting of tolylene 2,4- and 2,6-diisocyanate, diphenylmethane 4,4'- and 2,4'- and 2,2'-diisocyanate and polyphenylpolymethylene polyisocyanate ("polycyclic MDI").

11. Rigid PUR/PIR foam obtainable by a process according to any of Claims 1 to 10.

12. Rigid PUR/PIR foam according to Claim 11, **characterized in that** the apparent density is 25.0 to 300.0 kg/$m^3$, preferably 25.0 to 80.0 kg/$m^3$, particularly preferably 30.0 to 65.0 kg/$m^3$ and especially 30.0 to 45.0 kg/$m^3$.

13. Use of a rigid PUR/PIR foam according to either of Claims 11 or 12 as an insulating material or composite element with flexible or non-flexible outer layers.

14. Mixture containing a triurethane triol **A1.1** and a compound **A1.2, characterized in that** the compound **A1.2** is selected from the group consisting of polyether polyol, polyester polyol, polyether carbonate polyol and polyether ester polyol, wherein the triurethane triol **A1.1** has a structure of formula (II)

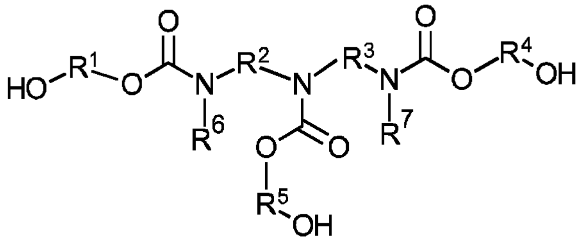

(II)

where

$R^1$ is linear or branched $C_2$- to $C_{24}$-alkylene which may optionally be interrupted by heteroatoms such as O, S or N and may be substituted, preferably $CH_2$-$CH_2$ or $CH_2$-$CH(CH_3)$,
$R^2$ is linear or branched $C_2$- to $C_{24}$-alkylene, $C_3$- to $C_{24}$-cycloalkylene, $C_4$- to $C_{24}$-arylene, $C_5$- to $C_{24}$-aralkylene, $C_2$- to $C_{24}$-alkenylene, $C_2$- to $C_{24}$-alkynylene, each of which may optionally be interrupted by heteroatoms such as O, S or N and/or each of which may be substituted by alkyl, aryl and/or hydroxyl, preferably $C_2$- to $C_{24}$-alkylene,
$R^3$ is linear or branched $C_2$- to $C_{24}$-alkylene, $C_3$- to $C_{24}$-cycloalkylene, $C_4$- to $C_{24}$-arylene, $C_5$- to $C_{24}$-aralkylene, $C_2$- to $C_{24}$-alkenylene, $C_2$- to $C_{24}$-alkynylene, each of which may optionally be interrupted by heteroatoms such as O, S or N and/or
$R^4$ each of which may be substituted by alkyl, aryl and/or hydroxyl, preferably $C_2$- to $C_{24}$-alkylene, is linear or branched $C_2$- to $C_{24}$-alkylene which may optionally be interrupted by heteroatoms such as O, S or N and may be substituted, preferably $CH_2$-$CH_2$ or $CH_2$-$CH(CH_3)$,
$R^5$ is linear or branched $C_2$- to $C_{24}$-alkylene which may optionally be interrupted by heteroatoms such as O, S or N and may be substituted, preferably $CH_2$-$CH_2$ or $CH_2$-$CH(CH_3)$,
$R^6$ is H, linear or branched $C_1$- to $C_{24}$-alkyl, $C_3$- to $C_{24}$-cycloalkyl, $C_4$- to $C_{24}$-aryl, $C_5$- to $C_{24}$-aralkyl, $C_2$- to $C_{24}$-alkenyl, $C_2$- to $C_{24}$-alkynyl, each of which may optionally be interrupted by heteroatoms such as O, S or N and/or each of which may be substituted by alkyl, aryl and/or hydroxyl, preferably H,
$R^7$ is H, linear or branched $C_1$- to $C_{24}$-alkyl, $C_3$- to $C_{24}$-cycloalkyl, $C_4$- to $C_{24}$-aryl, $C_5$- to $C_{24}$-aralkyl, $C_2$- to

$C_{24}$-alkenyl, $C_2$- to $C_{24}$-alkynyl, each of which may optionally be interrupted by heteroatoms such as O, S or N and/or each of which may be substituted by alkyl, aryl and/or hydroxyl, preferably H,

and wherein $R^1$ to $R^7$ may be identical or different from one another.

## Revendications

1. Procédé de fabrication de mousses rigides de PUR/PIR, contenant

**A1** un composant réactif vis-à-vis des isocyanates
**A2** un retardateur de flamme
**A3** un agent porogène
**A4** un catalyseur
**A5** éventuellement des adjuvants et additifs
**B** un composant polyisocyanate organique
**caractérisé en ce que**
le composant **A1** contient un tri-uréthane-triol **A1.1** et un composé **A1.2** choisi dans le groupe consistant en un polyétherpolyol, un polyesterpolyol, un polyéthercarbonatepolyol et un polyétheresterpolyol, le tri-uréthane-triol **A1.1** ayant une structure selon la formule (II)

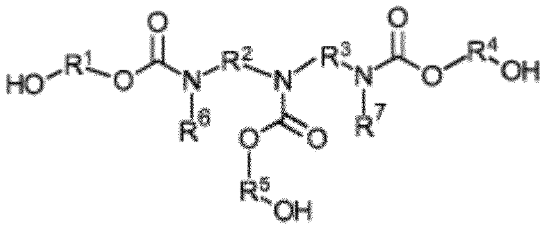

(II)

dans laquelle

$R^1$ représente un alkylène en $C_2$ à $C_{24}$ linéaire ou ramifié, qui peut éventuellement être interrompu par des hétéroatomes tels que O, S ou N et peut être substitué, de préférence $CH_2$-$CH_2$ ou $CH_2$-$CH(CH_3)$,
$R^2$ représente un alkylène en $C_2$ à $C_{24}$ linéaire ou ramifié, un cycloalkylène en $C_3$ à $C_{24}$, un arylène en $C_4$ à $C_{24}$, un aralkylène en $C_5$ à $C_{24}$, un alcénylène en $C_2$ à $C_{24}$, un alcynylène en $C_2$ à $C_{24}$, dont chacun peut être éventuellement interrompu par des hétéroatomes tels que O, S ou N, et dont chacun peut être substitué par un alkyle, un aryle et/ou un hydroxyle, de préférence un alkylène en $C_2$ à $C_{24}$,
$R^3$ représente un alkylène en $C_2$ à $C_{24}$ linéaire ou ramifié, un cycloalkylène en $C_3$ à $C_{24}$, un arylène en $C_4$ à $C_{24}$, un aralkylène en $C_5$ à $C_{24}$, un alcénylène en $C_2$ à $C_{24}$, un alcynylène en $C_2$ à $C_{24}$, dont chacun peut être éventuellement interrompu par des hétéroatomes tels que O, S ou N, et/ou chacun peut être substitué par un alkyle, un aryle et/ou un hydroxyle, de préférence un alkylène en $C_2$ à $C_{24}$,
$R^4$ représente un alkylène en $C_2$ à $C_{24}$ linéaire ou ramifié, qui peut être éventuellement interrompu par des hétéroatomes tels que O, S ou N et peut être substitué, de préférence $CH_2$-$CH_2$ ou $CH_2$-$CH(CH_3)$,
$R^5$ représente un alkylène en $C_2$ à $C_{24}$ linéaire ou ramifié, qui peut être éventuellement interrompu par des hétéroatomes tels que O, S ou N et peut être substitué, de préférence $CH_2$-$CH_2$ ou $CH_2$-$CH(CH_3)$,
$R^6$ représente H, un alkyle en $C_1$ à $C_{24}$ linéaire ou ramifié, un cycloalkyle en $C_3$ à $C_{24}$, un aryle en $C_4$ à $C_{24}$, un aralkyle en $C_5$ à $C_{24}$, un alcényle en $C_2$ à $C_{24}$, un alcynyle en $C_2$ à $C_{24}$, dont chacun peut être éventuellement interrompu par des hétéroatomes tels que O, S ou N et/ou chacun peut être substitué par un alkyle, un aryle et/ou un hydroxyle, de préférence H,
$R^7$ représente H, un alkyle en $C_1$ à $C_{24}$ linéaire ou ramifié, un cycloalkyle en $C_3$ à $C_{24}$, un aryle en $C_4$ à $C_{24}$, un aralkyle en $C_5$ à $C_{24}$, un alcényle en $C_2$ à $C_{24}$, un alcynyle en $C_2$ à $C_{24}$, dont chacun peut être éventuellement interrompu par des hétéroatomes tels que O, S ou N et/ou chacun peut être substitué par un alkyle, un aryle et/ou un hydroxyle, de préférence H,
et $R^1$ à $R^7$ pouvant être identiques ou différents les uns des autres.

2. Procédé selon la revendication 1, **caractérisé en ce que** le composant réactif vis-à-vis des isocyanates **A1** contient un composé **A1.2** ayant un indice d'hydroxyle de 100 à 550 mg KOH/g, de préférence de 100 à 450 mg KOH/g,

d'une manière particulièrement préférée de 100 à 350 mg KOH/g.

3. Procédé selon la revendication 1 ou 2, **caractérisé en ce que** le composant réactif vis-à-vis des isocyanates **A1** contient un composé **A1.2** ayant une fonctionnalité OH de 1,5 à 4,0, de préférence de 1,8 à 3,0, d'une manière particulièrement préférée de 1,9 à 2,5.

4. Procédé selon l'une des revendications 1 à 3, **caractérisé en ce que** le composé **A1.2** est un polyesterpolyol ou un polyétherpolyol, de préférence un polyesterpolyol.

5. Procédé selon l'une des revendications 1 à 4, **caractérisé en ce que** le composé **A1.2** est un polyesterpolyol pouvant être obtenu par la réaction d'un acide dicarboxylique aliphatique et/ou aromatique avec au moins un diol aliphatique.

6. Procédé selon l'une des revendications 1 à 5, **caractérisé en ce que** le tri-uréthane-triol **A1.1** peut être obtenu par la réaction entre du carbonate d'éthylène cyclique et/ou du carbonate de propylène cyclique avec un composé contenant au moins trois groupes amino, au moins deux des groupes amino étant des groupes amino primaires.

7. Procédé selon l'une des revendications 1 à 6, **caractérisé en ce que** la proportion du tri-uréthane-triol **A1.1** est de 0,1 à 35,0 % en poids, de préférence de 3,0 à 25,0 % en poids, d'une manière particulièrement préférée de 5,0 à 15,0 % en poids, par rapport à la somme des masses des composants **A1.1** et **A1.2**.

8. Procédé selon l'une des revendications 1 à 7, **caractérisé en ce que** l'indice d'isocyanate est de 100 à 500, de préférence de 180 à 450.

9. Procédé selon l'une des revendications 1 à 8, **caractérisé en ce que** le retardateur de flamme **A2** est présent selon une proportion de 2,0 % en poids à 30,0 % en poids, de préférence de 4,0 % en poids à 25,0 % en poids, d'une manière particulièrement préférée de 8,0 % en poids à 20,0 % en poids, par rapport au poids des composants **A1** à **A5.**

10. Procédé selon l'une des revendications 1 à 9, **caractérisé en ce qu'**on utilise en tant que composant polyisocyanate **B** au moins un composé choisi dans le groupe consistant en le diisocyanate de 2,4- et de 2,6-toluylène, le diisocyanate de 4,4'- et de 2,4'- et de 2,2'-diphénylméthane et le polyisocyanate de polyphénylpolyméthylène (« MDI à plusieurs noyaux).

11. Mousse rigide de PUR/PIR pouvant être obtenue par un procédé selon l'une des revendications 1 à 10.

12. Mousse rigide de PUR/PIR selon la revendication 11, **caractérisée en ce que** la masse volumique apparente est de 25,0 à 300,0 kg/$m^3$, de préférence de 25,0 à 80,0 kg/$m^3$, d'une manière particulièrement préférée de 30,0 à 65,0 kg/$m^3$ et en particulier de 30,0 à 45,0 kg/$m^3$.

13. Utilisation d'une mousse rigide de PUR/PIR selon l'une des revendications 11 ou 12 en tant que matériau isolant ou élément composite avec des couches de couverture souples ou non souples.

14. Mélange contenant un tri-uréthane-triol **A1.1** et un composé **A1.2**, **caractérisé en ce que** le composé **A1.2** est choisi dans le groupe constitué d'un polyétherpolyol, d'un polyesterpolyol, d'un polyéthercarbonatepolyol et d'un polyétheresterpolyol, le tri-uréthane-triol **A1.1** ayant une structure selon la formule (II)

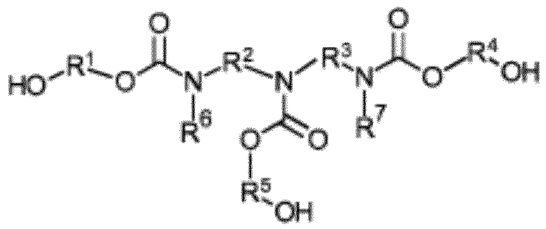

(II)

dans laquelle

$R^1$ représente un alkylène en $C_2$ à $C_{24}$ linéaire ou ramifié, qui peut éventuellement être interrompu par des

hétéroatomes tels que O, S ou N et peut être substitué, de préférence $CH_2$-$CH_2$ ou $CH_2$-$CH(CH_3)$,
$R^2$ représente un alkylène en $C_2$ à $C_{24}$ linéaire ou ramifié, un cycloalkylène en $C_3$ à $C_{24}$, un arylène en $C_4$ à $C_{24}$, un aralkylène en $C_5$ à $C_{24}$, un alcénylène en $C_2$ à $C_{24}$, un alcynylène en $C_2$ à $C_{24}$, dont chacun peut être éventuellement interrompu par des hétéroatomes tels que O, S ou N, et dont chacun peut être substitué par un alkyle, un aryle et/ou un hydroxyle, de préférence un alkylène en $C_2$ à $C_{24}$,
$R^3$ représente un alkylène en $C_2$ à $C_{24}$ linéaire ou ramifié, un cycloalkylène en $C_3$ à $C_{24}$, un arylène en $C_4$ à $C_{24}$, un aralkylène en $C_5$ à $C_{24}$, un alcénylène en $C_2$ à $C_{24}$, un alcynylène en $C_2$ à $C_{24}$, dont chacun peut être éventuellement interrompu par des hétéroatomes tels que O, S ou N, et/ou chacun peut être substitué par un alkyle, un aryle et/ou un hydroxyle, de préférence un alkylène en $C_2$ à $C_{24}$,
$R^4$ représente un alkylène en $C_2$ à $C_{24}$ linéaire ou ramifié, qui peut être éventuellement interrompu par des hétéroatomes tels que O, S ou N et peut être substitué, de préférence $CH_2$-$CH_2$ ou $CH_2$-$CH(CH_3)$,
$R^5$ représente un alkylène en $C_2$ à $C_{24}$ linéaire ou ramifié, qui peut être éventuellement interrompu par des hétéroatomes tels que O, S ou N et peut être substitué, de préférence $CH_2$-$CH_2$ ou $CH_2$-$CH(CH_3)$,
$R^6$ représente H, un alkyle en $C_1$ à $C_{24}$ linéaire ou ramifié, un cycloalkyle en $C_3$ à $C_{24}$, un aryle en $C_4$ à $C_{24}$, un aralkyle en $C_5$ à $C_{24}$, un alcényle en $C_2$ à $C_{24}$, un alcynyle en $C_2$ à $C_{24}$, dont chacun peut être éventuellement interrompu par des hétéroatomes tels que O, S ou N et/ou chacun peut être substitué par un alkyle, un aryle et/ou un hydroxyle, de préférence H,
$R^7$ représente H, un alkyle en $C_1$ à $C_{24}$ linéaire ou ramifié, un cycloalkyle en $C_3$ à $C_{24}$, un aryle en $C_4$ à $C_{24}$, un aralkyle en $C_5$ à $C_{24}$, un alcényle en $C_2$ à $C_{24}$, un alcynyle en $C_2$ à $C_{24}$, dont chacun peut être éventuellement interrompu par des hétéroatomes tels que O, S ou N et/ou chacun peut être substitué par un alkyle, un aryle et/ou un hydroxyle, de préférence H,
et $R^1$ à $R^7$ pouvant être identiques ou différents les uns des autres.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3098251 A1
- EP 2910585 A1
- EP 1359177 A1
- WO 2010043624 A
- EP 1923417 A
- EP 0007502 A1
- US 2764565 A


### In der Beschreibung aufgeführte Nicht-Patentliteratur


- **B. INOUE et al.** Copolymerization of Carbon Dioxide and Epoxide with Organometallic Compounds. *Die Makromolekulare Chemie,* 1969, vol. 130, 210-220
- **VON IONESCU.** Chemistry and Technology of Polyols for Polyurethanes. Rapra Technology Limited, 2005, 55
- Oligo-Polyols for Elastic Polyurethane. 263
- Polyester Polyols for Elastic Polyurethanes. 321
- Polyether Polyols for Rigid Polyurethane Foams. 419
- Polyester Polyols for Rigid Polyurethane Foams
- **VON W. SIEFKEN.** *Justus Liebigs Annalen der Chemie,* vol. 562, 75-136
- Kunststoff-Handbuch. Carl Hanser Verlag, 1993, vol. VII, 267
- Polyurethan Taschenbuch. Carl Hanser Verlag, 2001, 83-102